# EUROPÄISCHE PATENTSCHRIFT

(11) **EP 0 711 274 B1**
(45) Veröffentlichungstag und Bekanntmachung des Hinweises auf die Patenterteilung: **06.05.1998**
(21) Anmeldenummer: 94924726.6
(22) Anmeldetag: 08.07.1994
(51) Int. Cl.: C07C 239/20

(54) **VERFAHREN ZUR HERSTELLUNG VON O-SUBSTITUIERTEN HYDROXYLAMMONIUMSALZEN**
PROCESS FOR PREPARING O-SUBSTITUTED HYDROXYLAMMONIUM
PROCEDE DE PREPARATION DE SELS D'HYDROXYLAMMONIUM SUBSTITUES PAR OXYGENE

(30) Priorität: 31.07.1993 DE 4325851
(43) Veröffentlichungstag der Anmeldung: 15.05.1996
(73) Patentinhaber: BASF AKTIENGESELLSCHAFT, 67056 Ludwigshafen (DE)
(72) Erfinder: KLEIN, Ulrich, D-67117 Limburgerhof (DE); BUSCHMANN, Ernst, D-67069 Ludwigshafen (DE); KEIL, Michael, D-67251 Freinsheim (DE); GÖTZ, Norbert, D-67547 Worms (DE); HARREUS, Albrecht, D-67063 Ludwigshafen (DE)
(86) Internationale Anmeldenummer: EP9402239
(87) Internationale Veröffentlichungsnummer: WO9504032

(56) Entgegenhaltungen:
- EP-A- 0 259 850
- EP-A- 0 591 798
- CHEMICAL ABSTRACTS, vol. 109, no. 2, 11. Juli 1988, Columbus, Ohio, US; abstract no. 8922s, S. LETZ ET AL. Seite 139 ; & CS,A,243 595

## Beschreibung

Die vorliegende Erfindung betrifft ein verbessertes Verfahren zur Herstellung von O-substituierten Hydroxylammoniumsalzen der allgemeinen Formel I,

R¹-CHX-O-NH₂ · HL (I)

in der L für Halogen oder Hydrogensulfat, X für Wasserstoff oder C₁-C₄-Alkyl und R¹ für gewünschtenfalls substituiertes Phenyl, Thienyl, Furanyl oder Pyrrolyl oder für einen Rest -CR²=CR³R⁴ stehen, wobei R², R³ und R⁴ unabhängig voneinander Wasserstoff, Halogen oder C₁-C₄-Alkyl bedeuten,
durch Umsetzung eines Acetonoximethers der Formel II 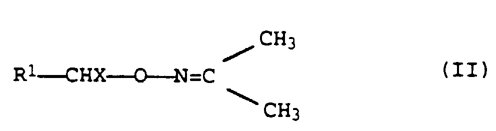 mit Wasser und einer Mineralsäure H-L unter laufender Entfernung des hierbei entstehenden Acetons.

O-substituierte Hydroxylamine lassen sich u.a. durch saure Hydrolyse von O-substituierten Acetonoximethern bei Siedetemperatur herstellen. So ist beispielsweise aus der DE-A 36 31 071 ein Verfahren zur Hydrolyse von Acetonoximethern vom Typ der Verbindungen II mit Salzsäure bekannt, das in einer kontinuierlich betriebenen Reaktionskolonne durchgeführt wird. Hierfür ist jedoch eine teure Spezialapparatur erforderlich.

Wesentlich wirtschaftlicher sind dagegen Verfahren, die in Standardapparaturen durchgeführt werden können:

So stellten Behrend et. al. (Liebigs Ann. Chem. 257, 203 (1890)), Benzyloxyamin-Hydrochlorid mit 50 % Ausbeute her.

Borek et. al. (J. Am. Chem. Soc. 58, 2020 (1936)) synthetisierten Carboxymethylenoxyamin-Hydrochlorid in 50 % Ausbeute.

Holland et. al. (J. Chem. Soc. 1948, 182) erhielten Diethylaminoethylenoxyamin nach dieser Methode, wobei sie jedoch keine Ausbeute angeben.

Brossi et. al. (Heterocycles 20, 839 (1983)) erhielten 3'-(2,4,5-Trichlorphenoxy)propyloxyamin-Hydrochlorid durch Hydrolyse des entsprechenden Acetonoximethers in ethanolischer Salzsäure. Die Ausbeute betrug jedoch nur 47 %.

Nachteilig bei diesen bekannten Verfahren ist allerdings die unbefriedigende Reinheit der Verfahrensprodukte. Die störenden Nebenprodukte resultieren hauptsächlich aus der teilweisen Zersetzung der gewünschten Hydroxylamin-Derivate unter den geforderten Reaktionsbedingungen (vgl. hierzu auch Zech und Metzger in Houben-Weyl, Methoden der Organischen Chemie, Bd. 10/1, 4. Auflage 1971, S. 1186). Von dem zusätzlichen Reinigungsschritt abgesehen sind die Verfahren auch wegen der geringen Ausbeute an Wertprodukt technisch wenig rentabel.

In der älteren deutschen Anmeldung DE-A 42 33 333 wird ein Verfahren zur Synthese von O-Alkylhydroxylammoniumsalzen mit kurzkettigen Alkylresten beschrieben. Die Hydrolyse des Acetonoximethers erfolgt hier mit konzentrierter Salzsaure in einem Zwei-Phasen-System mit unpolaren Lösungsmitteln wie Xylol, Toluol und Cyclohexan. Dieses Verfahren eignet sich allerdings nur für die Synthese von relativ polaren O-Alkylhydroxylammoniumsalzen. Es eignet sich nicht für die Synthese der weniger polaren Verbindungen II, die sich in dem Lösungsmittel lösen würden. Die Reaktionszeiten werden in diesem Fall durch Phasenübergang sehr lang.

Aus Org. Synth., Coll. Vol. 3, S. 172, ist die Hydrolyse von Acetonoxim-O-(methoxycarbonylmethylen)ether mit wässriger Salzsäure unter gleichzeitiger destillativen Entfernung des Nebenproduktes Aceton bekannt. Die Hydrolyse erfolgt bei 100°C. Die Ausbeuten liegen bei 66 - 72 %. Diese Methode ist jedoch zur Synthese der Verbindungen I ebenfalls nicht geeignet, da die O-Allyl- und O-Benzyl-substituierten Hydroxylammoniumsalze I bei Temperaturen über 50 °C nicht stabil sind. Durch Spaltung der N-O-Bindung würde als Nebenprodukt Ammoniumchlorid entstehen, das in einem nachfolgenden Reinigungschritt erst wieder abgetrennt werden müßte. Bei Temperaturen oberhalb von 100 °C kann die Zersetzung der Verbindungen II sogar explosionsartig erfolgen. Eine hohe Ausbeute an I ist nach diesem Verfahren jedenfalls nicht zu erzielen.

Aufgabe der vorliegenden Erfindung war es demnach, ein verbessertes Verfahren bereitzustellen, mit dem die O-substituierten Hydroxylammoniumsalze I in guter Ausbeute und mit sehr hoher Reinheit hergestellt werden können.

Demgemäß wurde das vorliegende Verfahren gefunden, das dadurch gekennzeichnet ist, daß man die Hydrolyse diskontinuierlich bei 0 - 50 °C und unter 10 - 500 mbar Druck durchführt.

Besonders vorteilhaft arbeitet man bei einer Reaktionstemperatur von 30 bis 50 °C und einem Druck von 40 bis 50 mbar.

Nach bisherigen Erkenntnissen ist es zweckmäßig, pro Moläquivalent Acetonoximether II etwa 2 Moläquivalente an Mineralsäure einzusetzen. Ein größerer Überschuß an H-L ist möglich, bringt jedoch keine Vorteile.

Als Mineralsäuren H-L kommen insbesondere Salzsäure und Schwefelsäure in Betracht, wobei konzentrierte Salzsäure besonders bevorzugt ist.

Das vorliegende Verfahren wird vorteilhaft in wässriger Phase durchgeführt, wobei auch ein inertes, mit Wasser gut mischbares Lösungmittel zugesetzt werden kann, z.B. ein niederer Alkohol wie Methanol, Ethanol und Propanol, oder eine kurzkettige Carbonsäure wie Essigsäure und Propionsäure.

Die Mengen an Lösungsmittel und Mineralsäure H-L sind nicht kritisch. Normalerweise arbeitet man mit einem 5 bis 100fachen molaren Überschuß an Wasser und einem 30 - 200 %igen molaren Überschuß an H-L, bezogen auf den Acetonoximether II.

Das Fortschreiten der Reaktion kann mittels üblicher analytischer Methoden wie Dünnschichtchromatographie, Hochdruckflüssigkeitschromatographie und Gaschromatographie, verfolgt werden.

Nach beendeter Unsetzung und weitgehender Entfernung des Hydrolyse-Nebenproduktes Aceton, üblicherweise destillativ, entfernt man das restliche Wasser und die überschüssige Mineralsäure H-L, zweckmäßigerweise durch azeotrope Destillation. Als Wasserschlepper eignen sich hierzu allgemein alle organischen Lösungsmittel, die mit Wasser Azeotrope bilden, also beispielsweise Benzol, Toluol, Xylol, Pentan, Hexan, Heptan, Cyclohexan und Cycloheptan.

Normalerweise kristallisiert das Verfahrensprodukt I (H-L-Salz) aus dem Rückstand aus und kann dann mittels üblicher Methoden wie Filtration oder Extraktion (z.B. mit Wasser oder Alkoholen) isoliert und gewünschtenfalls weiter gereinigt werden, z.B. durch Kristallisation, Rektifikation oder mittels chromatographischer Methoden.

Die als Ausgangsstoffe dienenden Acetonoximether II sind bekannt oder auf bekannte Weise erhältlich (vgl. z.B. Houben-Weyl, Methoden der Organischen Chemie, Band X/4, Georg Thieme Verlag, Stuttgart 1968, Seiten 217ff).

Das Verfahren laßt sich mit Erfolg zur Synthese aller definitionsgemäßen O-substituierten Hydroxylammoniumsalze I anwenden, vor allem auf solche Verbindungen, bei denen die Variablen die folgende Bedeutung haben:
L Chlor, Brom oder Hydrogensulfat, insbesondere Chlor;
X Wasserstoff oder C₁-C₄-Alkyl, insbesondere Wasserstoff;
R¹
   - Phenyl, Thienyl, Furanyl oder Pyrrolyl, die jeweils unsubstituiert sein oder ein bis drei Halogenatome und/ oder C₁-C₄-Alkyl-Reste, insbesondere Fluor, Chlor, Brom, Methyl oder Ethyl, tragen können;
   - einen Rest -CR²=CR³R⁴, wobei R², R³ und R⁴ jeweils Wasser> stoff, Halogen wie Fluor, Chlor oder Brom, oder C₁-C₄-Alkyl, insbesondere Chlor oder Methyl, bedeuten;
besonders bevorzugt sind Phenyl, Halogenphenyl, Thienyl und -CH=CH-Cl, wobei -CH=CH-Cl ganz besonders bevorzugt ist.

Im Hinblick auf die Verwendung der O-substituierten Hydroxylammoniumsalze I als Zwischenprodukte für Pflanzenschutzmittel und Pharmaka, insbesondere zur Herstellung von herbiziden Cyclohexenonoximethern (vgl. z.B. EP-A 136 647, EP-A 136 702 und EP-A 142 741), ist die Herstellung von O-Benzylhydroxylammoniumsalzen aus Acetonoxim-O-benzylether und insbesondere von E-(O)-(3-Chlor-2-propenyl)-hydroxylammoniumsalzen aus E-Acetonoxim-O-(3-chlor-2-propenyl)ether ganz besonders bevorzugt.

### Herstellungsbeispiele

### Beispiel 1 (erfindungsgemäß): O-Benzylhydroxylamin-Hydrochlorid

Man destillierte eine Mischung aus 198,6 g (1,0 Mol) Acetonoxim-O-benzylether (Reinheit: 82 %) und 200 g (2,0 Mol) 36 gew.-%iger Salzsaure bei 50 °C Sumpftemperatur, 40 - 35 mbar Druck, und einem Rücklaufverhältnis von 6 über eine 30 cm lange Kolonne, die mit Glas-Raschigringen gefüllt war. Nach Abdestillieren von ca. 60 g eines Gemisches aus verdünnter Salzsäure und Aceton versetzte man den Rückstnand mit 500 ml Toluol. Anschließend wurden bei 50 °C Sumpftemperatur und 20 - 30 mbar Druck noch 120 g Salzsäure abdestilliert. Das feste O-Benzylhydroxylamin-Hydrochlorid wurde abgetrennt und bei 50 °C unter vermindertem Druck getrocknet. Ausbeute: 172,5 g (94 %; 88 %ige Reinheit)

### Beispiel 2 (erfindungsgemäß):

### E-(O)-(3-Chlor-2-propenyl)hydroxyl-amin-Hydrochlorid

Man destillierte eine Mischung aus 158,3 g (1,0 Mol) (E)-Acetonoxim-O-(3-Chlor-2-propenyl)ether (Reinheit: 91 %) und 200 g (2,0 Mol) 36 gew.-%ige Salzsäure bei 40 - 50 °C Sumpftemperatur, 40 - 50 mbar Druck, einem Rücklaufverhältnis von 6, über eine 30 cm lange, mit Glas-Raschigringen gefüllte Kolonne. Innerhalb von 15 Stunden wurden 145 g eines Gemisches aus verdünnter Salzsäure und Aceton abdestilliert. Während der Destillation kristalliserte das Hydroxylamin-Hydrochlorid aus dem Reaktionsgemisch aus. Nach Zugabe von 500 ml Cyclohexan wurde bei 40 - 45 °C und 250 - 240 mbar Druck die restliche Salzsäure azeotrop abdestilliert, wonach man das feste E-(O)-(3-Chlor-2-propenyl)hydroxylamin-Hydrochlorid abtrennte und 12 Std. unter vermindertem Druck bei 50 °C trocknete. Der Rückstand enthielt noch 14,5 g (0,1 Mol) E-Acetonoxim-O-(3-chlor-2-propenyl)ether. Ausbeute: 116 g (90 %; 98 %ige Reinheit); Smp.: 175 - 176 °C.

### Beispiel 3 (erfindungsgemäß):

### E-(O)-(3-Chlor-2-propenyl)hydroxyl-amin-hydrochlorid

Aus einer Mischung aus 23,0 kg (144,8 Mol) Acetonoxim-O-(3-Chlor-2-propenyl)ether (Reinheit: 91 %) und 30,0 kg (205 Mol) 36 gew.-%iger Salzsäure wurden bei einer Sumpftemperatur von 45 - 49 °C, einem Druck von 40 - 50 mbar und einem Rücklaufverhältnis von 6 ein Gemisch aus verdünnter Salzsäure und Aceton abdestilliert. Zur Aufarbeitung wurde die Reaktionsmischung mit 45 1 Toluol versetzt, wonach man 43 °C und 50 - 60 mbar Druck die restliche Salzsäure azeotrop abdestillierte. Das als Rohprodukt verbliebene E-(O)-(3-Chlor-2-propenyl)hydroxylamin-Hydrochlorid wurde abgetrennt und bei 50 °C unter vermindertem Druck getrocknet. Ausbeute: 16,0 kg (75 %; 98 %ige Reinheit); Smp.: 176 - 178°C.

| Elementaranalyse: | | | | | |
|---|---|---|---|---|---|
| Ber. | C: 25,02; | H: 5,00; | O: 11,11; | N: 9,73; | Cl: 49,24; |
| Gef. | C: 24,90; | H: 4,90; | O: 11,80; | N: 9,70; | Cl: 48,80. |

### Beispiel 4 (Vergleichsbeispiel, analog zu Org. Synth., Coll. Vol. 3, S. 172): E-(O)-(3-Chlor-2-propenyl)-hydroxylamin-hydrochlorid

Eine Mischung aus 158,3 g (1,0 Mol) 91 %igem (E)-Acetonoxim-O-(3-chlor-2-propenyl)ether und 200 g (2,0 Mol) 36 gew.-%iger Salzsäure wurde bei 68 - 69 °C Sumpftemperatur, bei 165 - 200 mbar, einem Rücklaufverhältnis von 16 über eine 30 cm Kolonne, die mit Glas-Raschigringen gefüllt ist, destilliert. Innerhalb von 15 Std. erhielt man so 145 g einer Mischung aus verdünnter Salzsäure und Aceton. Aus dem Rückstand kristallisierte das Hydroxylamin-Hydrochlorid aus. Zur Aufarbeitung versetzte man den Rückstand mit 500 ml Cyclohexan und destilliert bei 40 - 45 °C und 250 - 240 mbar die restliche Salzsäure azeotrop ab.

Das feste E-(O)-(3-Chlor-2-propenyl)hydroxylamin-Hydrochlorid wurde abgetrennt und 12 Std bei 50 °C unter vermindertem Druck getrocknet. Ausbeute: 100 g (69 %; 80 %ige Reinheit); Smp.: 160 - 162 °C.

Gemäß ¹H-NMR-Spektrum enthielt das Produkt noch 20 gew.-% Ammoniumchlorid.

### Beispiel 5 (Vergleichsbeispiel, gemäß DE-A 42 33 333): E-(O)-(3-Chlor-2-propenyl)-hydroxylammoniumchlorid

Eine Mischung aus 73,8 g (0,5 Mol) E-Acetonoxim-O-(3-chlor-2-propenyl)ether, 1 1 Cyclohexan und 76 g (0,75 Mol) 36 gew.-%iger Salzsäure wurde bei einer Sumpftemperatur von 72 - 75 °C, einem Rücklaufverhältnis von 6, über eine Kolonne mit 30 cm Länge, die mit Glas-Raschigringen gefüllt ist, destilliert, wobei während der Destillation weitere 1900 ml Cyclohexan und weitere 150 g (1,5 Mol) 36 gew-%ige Salzsäure zugegeben wurden. Nach 25 Std. hatten sich so 1935 g Destillat angesammelt. Aus dem Rückstand wurde die restliche Salzsäure nach Zugabe von Cyclohexan azeotrop abdestilliert, wonach man auskristallisiertes E-(O)-(3-Chlor-2-propenyl)hydroxylammoniumchlorid abtrennte. Ausbeute: 36,1 g (40 %; 80 %ige Reinheit); Smp.: 160-162 °C.

Gemäß ¹H-NMR-Spektrum enthielt das Produkt noch 20 gew.-% Ammoniumchlorid.

### Herstellung der Vorstufen:

### Acetonoxim-O-benzylether

Zu einer Lösung von 352 g (8,8 mol) Natriumhydroxid in 2 kg Wasser wurden in einem 4 l Reaktionsgefäß 292 g (4,0 mol) Acetonoxim gegeben. Anschließend erwärmte man die Mischung auf 70-75 °C und versetzte sie mit 506 g (4,0 mol) Benzylchlorid. Nach 3 Stunden Rühren bei 75-80 °C wurde das Reaktionsgemisch abgekühlt, wonach die organische Phase abgetrennt und getrocknet wurde. Ausbeute: 579 g (73 %; 82 %ige Reinheit).

### E-Acetonoxim-O-(3-chlor-2-propenyl)ether

14,5 kg 25 gew.-%ige wässrige Hydroxylammoniumsulfatlösung (= 22 Mol Hydroxylammoniumsulfat) und 10 kg Wasser wurden in einem 50 l-Reaktionsgefaß nach und nach mit insgesamt 2,9 kg (50 Mol) Aceton und 2,5 1 50 gew.-%iger Natronlauge versetzt. Die Zugabe erfolgte so, daß der pH-Wert der Reaktionsmischung konstant zwischen 4,5 und 5,0 lag. Nach beendeter Zugabe wurde die Mischung noch zwei Std. gerührt und anschließend mit weiteren 5,28 kg (66 Mol) 50 gew.-%iger Natronlauge versetzt. Danach erwärmte man auf 70 °C und gab 5,09 kg (44 Mol) E-1,3-Dichlorpropen zu dem Reaktionsgemisch, wonach man drei Stunden bei 70 - 80 °C rührte. Durch Destillation bei einem Unterdruck von 140 mbar und einer Übergangstemperatur von 25 - 53 °C erhielt man ein zweiphasiges Destillat, dessen organische Phase das Produkt enthielt. Ausbeute: 5,0 kg (76 %; 91 %ige Reinheit).

## Patentansprüche

1. Verfahren zur Herstellung von O-substituierten Hydroxylammoniumsalzen der allgemeinen Formel I,
R¹-CHX-O-NH₂ · HL (I)
in der L für Halogen oder Hydrogensulfat, X für Wasserstoff oder C₁-C₄-Alkyl und R¹ für gewünschtenfalls substituiertes Phenyl, Thienyl, Furanyl oder Pyrrolyl oder für einen Rest -CR²=CR³R⁴ stehen, wobei R², R³ und R⁴ unabhängig voneinander Wasserstoff, Halogen oder C₁-C₄-Alkyl bedeuten, durch Umsetzung eines Acetonoximethers der Formel II 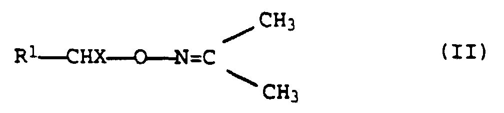 mit Wasser und einer Mineralsäure H-L unter laufender Entfernung des hierbei entstehenden Acetons, dadurch gekennzeichnet, daß man die Hydrolyse diskontinuierlich bei 0 - 50 °C und unter 10 - 500 mbar Druck durchführt.

2. Verfahren nach Anspruch 1, dadurch gekennzeichnet, daß man es auf die Herstellung von E-(O)-(3-chlor-2-propenyl)-hydroxylammoniumsalzen aus E-Acetonoxim-O-(3-chlor-2-propenyl)ether oder von O-Benzylhydroxylammoniumsalzen aus Acetonoxim-O-benzylether anwendet.

3. Verfahren nach Anspruch 1, dadurch gekennzeichnet, daß man es auf die Herstellung von E-(O)-(3-Chlor-2-propenyl)-hydroxylammoniumsalzen aus E-Acetonoxim-O-(3-chlor-2-propenyl)ether anwendet.

## Claims

1. A process for preparing O-substituted hydroxylammonium salts of the general formula I
R¹-CHX-O-NH₂ · HL (I)
where L is halogen or hydrogensulfate, X is hydrogen or C₁-C₄-alkyl and R¹ is phenyl, thienyl, furanyl or pyrrolyl which, if desired, are substituted or a radical -CR²=CR³R⁴, R², R³ and R⁴ independently of one another being hydrogen, halogen or C₁-C₄-alkyl, by reaction of an acetone oxime ether of the formula II 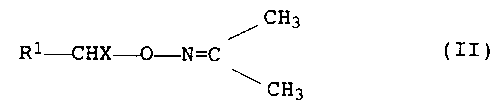 with water and a mineral acid H-L with continuous removal of the acetone formed in this process, which comprises carrying out the hydrolysis batchwise at 0 - 50°C and under a pressure of 10 - 500 mbar.

2. A process as claimed in claim 1, which is used for the preparation of E-(O)-(3-chloro-2-propenyl)hydroxylammonium salts from E-acetone oxime O-(3-chloro-2-propenyl) ether or of O-benzylhydroxylammonium salts from acetone oxime O-benzyl ether.

3. A process as claimed in claim 1, which is used for the preparation of E-(O)-(3-chloro-2-propenyl)hydroxylammonium salts from E-acetone oxime O-(3-chloro-2-propenyl) ether.

## Revendications

1. Procédé de préparation de sels d'hydroxylammonium substitués à l'oxygène et répondant à la formule générale I
R¹-CHX-O-NH₂ · HL (I)
dans laquelle L représente un halogène ou le groupe hydrogénosulfate, X représente l'hydrogène ou un groupe alkyle en C₁-C₄ et R¹ représente un groupe phényle, thiényle, furannyle ou pyrrolyle éventuellement substitué ou un groupe -CR²=CR³R⁴ dans lequel R², R³ et R⁴ représentent chacun, indépendamment les uns des autres, l'hydrogène, un halogène ou un groupe alkyle en C1-C4, par réaction d'un éther d'acétonoxime de formule II 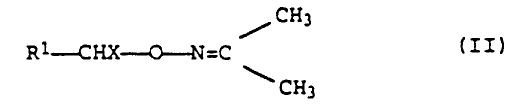 avec l'eau et un acide minéral H-L avec élimination en continu de l'acétone formée, caractérisé par le fait que l'on procède à l'hydrolyse en discontinu à des températures de 0 à 50°C et sous une pression de 10 à 500 mbar.

2. Procédé selon la revendication 1, caractérisé par le fait qu'on l'exploite pour la préparation des sels de E-(O)-(3-chloro-2-propényl)-hydroxylammonium de l'éther O-(3-chloro-2-propénylique) de la E-acétonoxime ou des sels d'O-benzylhydroxylammonium de l'éther O-benzylique de l'acétonoxime.

3. Procédé selon la revendication 1, caractérisé par le fait qu'on l'exploite pour la préparation des sels de E-(O)-(3-chloro-2-propényl)-hydroxylammonium de l'éther O-(3-chloro-2-propénylique) de l'E-acétonoxime.
